# EUROPEAN PATENT SPECIFICATION

(11) **EP 2 947 061 B1**
(45) Date of publication and mention of the grant of the patent: **19.02.2020**
(21) Application number: 14168854.9
(22) Date of filing: 19.05.2014
(51) Int. Cl.: C04B 35/119, C04B 35/44, A61L 27/10, C04B 35/111, C04B 35/80

(54) **Bioceramic component**
Biokeramische Komponente
Composant de biocéramique

(43) Date of publication of application: 25.11.2015
(73) Proprietor: BBL Technology Transfer GmbH & Co. KG, 90562 Heroldsberg (DE)
(72) Inventor: BILLAU, Karl, 91233 Neunkirchen (DE); KIEFER, Gundula, 73249 Wernau (DE); Dr. BURGER, Wolfgang, 73207 Plochingen (DE)
(74) Representative: Fleck, Hermann-Josef

(56) References cited:
- EP-A2- 2 684 555
- EP-B1- 3 071 522
- WO-A1-2008/040813
- WO-A1-2011/000390
- WO-A2-2006/070201
- US-A1- 2012 308 837
- JIN X ET AL: "Effects of powder preparation method on the microstructure and mechanical performance of ZTA/LaAl11O18 composites", JOURNAL OF THE EUROPEAN CERAMIC SOCIETY, ELSEVIER SCIENCE PUBLISHERS, BARKING, ESSEX, GB, vol. 24, no. 4, 1 April 2004 (2004-04-01), pages 653-659, XP004551082, ISSN: 0955-2219, DOI: 10.1016/S0955-2219(03)00257-7
- IPEK AKIN ET AL: "Effect of CeO2 addition on densification and microstructure of Al2O3-YSZ composites", CERAMICS INTERNATIONAL, ELSEVIER, AMSTERDAM, NL, vol. 37, no. 8, 23 May 2011 (2011-05-23), pages 3273-3280, XP028309316, ISSN: 0272-8842, DOI: 10.1016/J.CERAMINT.2011.05.123 [retrieved on 2011-05-27]
- F. KERN: "Effect of In Situ-Formed Cerium Hexaaluminate Precipitates on Properties of Alumina -24 Vol% Zirconia (1.4Y) Composites", JOURNAL OF CERAMIC SCIENCE AND TECHNOLOGY, vol. 4, no. 4, 5 August 2013 (2013-08-05), pages 177-186, XP009180895, Göller Verlag GmbH, Germany DOI: 10.4416/JCST2013-00014

## Description

The present invention relates to the use of a ceramic material with an alumina matrix and zirconia as a bioceramic component.

Bioceramic components of that type, namely artificial prostheses as e.g. a hip joint implant or a knee joint implant, are disclosed in WO 2011/083 023 A1 (EP 2 513 010 A1). The composite ceramic material described therein comprises dispersed zirconia wherein the tetragonal modification should be retained preferably by mechanical stabilization whereas chemical stabilizers such as Y₂O₃ are considered as prone to hydrothermal aging. As a requirement for the mechanical stabilization an aluminum oxide proportion of at least 65 % by volume and a zirconium oxide proportion of 10 to 35 % by volume is mentioned.

As a further relevant ceramic material established on the market EP 0 542 815 B1 describes zirconia and platelet reinforced ceramics the composition comprising 60 - 98 vol % of a matrix based on a alumina-chromia solid solution wherein that matrix is based on 67,1 - 99,2 vol % of Al₂O₃-Cr₂O₃ solid solution and 0,8 - 32,9 vol % SrAl₁₂ₓCrₓO₁₉ (x = 0,0007 - 0,045); moreover that composition comprises 2 - 40 vol % tetragonal zirconia which is stabilized with 0,2 - 3 mol % yttria or 10 - 15 mol % CeO₂, Pr₆O_{11,} Tb₂O₃.

DE 198 50 366 A1 also describes a platelet reinforced sintered material with related composition as described in EP 0 542 815 B1. However, platelet formation is based on different oxides.

WO 2008/040813 A1 discloses a sintered material comprising zirconia, alumina and rare earth aluminate platelets employable in various fields of application, including the medical field as a high strength and tough material for bridges in the orthodontic field, as a dental implant, as a hip, knee, shoulder, ankle and finger implant.

WO 2011/000390 A1 describes a ceramic material comprising a first phase including zirconia, yttrium and cerium, a second phase comprising alumina and a third phase comprising metal aluminate platelets, used as a roller bearing.

EP 3 071 522 B1 discloses a sintered ceramic material produced from a mixture of alumina, zirconia, yttria, ceria, lanthanum oxide and praseodymium oxide.

Xihai Jin et al., Journal of the European Ceramic Society 24 (2004) 653-659, disclose the effects of the powder preparation method on the microstructure and mechanical performances of ZTA/LaAl₁₁O₁₈ composites.

It is an object of the present invention to make available bioceramic components having improved material stability.

The object is achieved by the features of claim 1, wherein a ceramic material having the following composition:
- 55 - 90 vol % of an alumina matrix
- wherein 10 - 75 vol % of the alumina matrix form hexagonal platelets with the chemical composition of LaAl₁₁O₁₈,
- 10 - 45 vol % zirconia which always is co-stabilized by yttria in the amount of 0,5 - 2,5 mol % related to zirconia and ceria in the amount of 2 - 10 mol % related to zirconia,
- 0,1 - 1,0 vol % of Pr₆O₁₁ forming a solid solution with alumina and acting together with ceria as bridge oxide between alumina and zirconia,
wherein the content of alumina and zirconia together with their composite substances complete to 100% unless unvolontary contaminants ,
is used as a bioceramic component.

Examples for the bioceramic components of the present invention are ball-heads, inserts, femoral knee components, tibia parts, ankle joints, shoulder joints, finger joints or other implants or artificial prostheses.

In the composition of ceramic material in accordance with claim 1 the content of alumina and zirconia together with their composite substances complete to 100 % (unless unvoluntary contaminants).

The composition with the chemical co-stabilization taught in claim 1 yields a high stability of the bioceramic components. Stability is reinforced by the added ceria oxide stabilizing the metastable zirconium oxide phase and forming anisotropic crystallites with aluminum oxide wherein the process is directed to the formation of the crystallites.

Advantageous embodiments of the compositions are indicated in the dependent claims wherein
- 20 - 60 vol %, especially 33 - 50 vol % of the matrix are in the form of hexagonal platelets;
- the maximum amount of stabilizing yttria is 1 - 2 mol %, especially 1,5 - 1,8 mol %;
- the maximum amount of stabilizing ceria is 2,5 - 6 mol %, especially 3 - 5 mol %;
- the maximum amount of Pr₆O₁₁ is 0,2 - 0,8 vol %, especially 0,25 - 0,5 vol %.

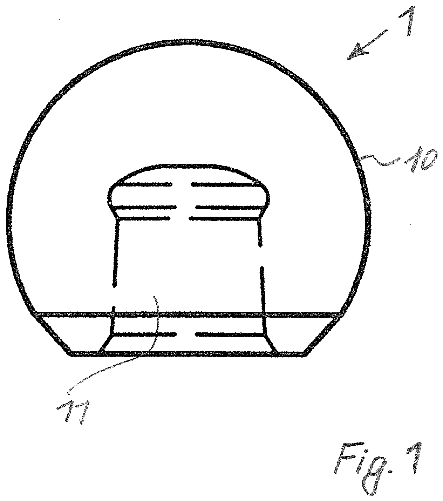

Fig. 1 shows an embodiment of a bioceramic component, namely a ball-head 1. The ball-head can be used as a part of a ball joint.

The ball-head 1 is bio-compatible and comprises a spherical segment having an outer spherical segment surface 10, to be fit in a spherical bearing of a patient's joint. In the inner portion of the ball-head 1 a hollow receptacle 11 is formed for fixing an extending part.

The ball-head 1 is made of a ceramic material with the composition described above. The surface 10 is polished and yields stable and excellent sliding properties whereas the receptacle 11 is designed to ensure stable connection of the extending part fitted therein e.g. by screwing, bonding and/or cementing.

The production of the composite ceramic material comprises as the essential steps:
- dispersing the components in water
- grinding and mixing in an agitator ball mill (attrition milling)
- addition of a binder
- spray drying the suspension
- pressing the powder mixture
- machining the pressed body by cutting methods to an approximately final shape
- presintering the bodies to closed porosity (approximately 95 - 96 % of the theoretical density)
- high-temperature isostatic redensification of the presintered body (HIPen)
- annealing the redensified components
- grinding and polishing the components to the final contour.

## Claims

1. Use of a ceramic material having the following composition:
- 55 - 90 vol % of an alumina matrix
- wherein 10 - 75 vol % of the alumina matrix form hexagonal platelets with the chemical composition of LaAl₁₁O₁₈,
- 10 - 45 vol % zirconia which always is co-stabilized by yttria in the amount of 0,5 - 2,5 mol % related to zirconia and ceria in the amount of 2 - 10 mol % related to zirconia,
- 0,1 - 1,0 vol % of Pr₆O₁₁ forming a solid solution with alumina and acting together with ceria as bridge oxide between alumina and zirconia,
wherein the content of alumina and zirconia together with their composite substances complete to 100% unless unvoluntary contaminants as a bioceramic component.

2. Use of a ceramic material as a bioceramic component according to claim 1, wherein 20 - 60 vol % of the matrix are in the form of the hexagonal platelets.

3. Use of a ceramic material as a bioceramic component according to claim 1 or 2, wherein 33 - 50 vol % of the matrix are in the form of the hexagonal platelets.

4. Use of a ceramic material as a bioceramic component according to one of the preceding claims,
wherein the maximum amount of stabilizing yttria is 1 - 2 mol %.

5. Use of a ceramic material as a bioceramic component according to one of the preceding claims,
wherein the maximum amount of stabilizing yttria is 1,5 - 1,8 mol %.

6. Use of a ceramic material as a bioceramic component according to one of the preceding claims,
wherein the maximum amount of stabilizing ceria is 2,5 - 6 mol %.

7. Use of a ceramic material as a bioceramic component according to one of the preceding claims,
wherein the maximum amount of stabilizing ceria is 3 - 5 mol %.

8. Use of a ceramic material as a bioceramic component according to one of the preceding claims,
wherein the maximum amount of Pr₆O₁₁ is 0,2 - 0,8 vol %.

9. Use of a ceramic material as a bioceramic component according to one of the preceding claims,
wherein the maximum amount of Pr₆O₁₁ is 0,25 - 0,5 vol %.

## Patentansprüche

1. Verwendung eines Keramikwerkstoffs mit folgender Zusammensetzung:
- 55-90 Vol.-% einer Aluminiummatrix
- wobei 10-75 Vol.-% der Aluminiummatrix als hexagonale Plättchen der chemischen Zusammensetzung La Al₁₁O₁₈ ausgebildet sind,
- 10-45 Vol.-% Zirkonium, das immer durch einen Anteil von 0,5-2,5 Vol.-% Yttrium bezogen auf Zirkonium und durch einen Anteil von 2-10 Vol.-% Cer bezogen auf Zirkonium mischstabilisiert ist,
- 0,1-1,0 Vol.-% Pr₆O₁₁, das eine feste Verbindung mit Aluminium bildet und zusammen mit Cer als Brückenoxid zwischen Aluminium und Zirkonium wirkt,
wobei der Anteil an Aluminium und Zirkonium zusammen mit ihren zusammengesetzten Substanzen sich bis auf unvermeidbare Verunreinigungen auf 100 Vol.-% ergänzen,
als biokeramische Komponente.

2. Verwendung eines Keramikwerkstoffs als biokeramische Komponente nach Anspruch 1,
wobei 20-60 Vol.-% der Matrix als hexagonale Plättchen ausgebildet sind.

3. Verwendung eines Keramikwerkstoffs als biokeramische Komponente nach Anspruch 1 oder 2,
wobei 33-50 Vol.-% der Matrix als hexagonale Plättchen ausgebildet sind.

4. Verwendung eines Keramikwerkstoffs als biokeramische Komponente nach einem der vorhergehenden Ansprüche,
wobei der maximale Anteil an stabilisierendem Yttrium 1-2 Mol.-% beträgt.

5. Verwendung eines Keramikwerkstoffs als biokeramische Komponente nach einem der vorhergehenden Ansprüche,
wobei der maximale Anteil an stabilisierendem Yttrium 1,5-1,8 Mol.-% beträgt.

6. Verwendung eines Keramikwerkstoffs als biokeramische Komponente nach einem der vorhergehenden Ansprüche,
wobei der maximale Anteil an stabilisierendem Cer 2,5-6 Mol.-% beträgt.

7. Verwendung eines Keramikwerkstoffs als biokeramische Komponente nach einem der vorhergehenden Ansprüche,
wobei der maximale Anteil an stabilisierendem Cer 3-5 Mol.-% beträgt.

8. Verwendung eines Keramikwerkstoffs als biokeramische Komponente nach einem der vorhergehenden Ansprüche,
wobei der maximale Anteil an Pr₆O₁₁ 0,2-0,8 Vol.-% beträgt.

9. Verwendung eines Keramikwerkstoffs biokeramische Komponente nach einem der vorhergehenden Ansprüche,
wobei der maximale Anteil an Pr₆O₁₁ 0,25-0,5 Vol.-% beträgt.

## Revendications

1. Utilisation d'un matériau céramique présentant la composition suivante :
- 55 à 90 % en volume d'une matrice d'alumine
- 10 à 75 % en volume de la matrice d'alumine formant des plaquettes hexagonales ayant la composition chimique de LaAl₁₁O₁₈,
- 10 à 45 % en volume de zircone qui est toujours co-stabilisée par l'yttria en une quantité de 0,5 à 2,5 % en moles par rapport à la zircone et par la cérine en une quantité de 2 à 10 % en moles par rapport à la zircone,
- 0,1 à 1,0 % en volume de Pr₆O₁₁ formant une solution solide avec de l'alumine et agissant conjointement avec la cérine comme pont d'oxyde entre l'alumine et la zircone,
la teneur en alumine et en zircone ainsi que leurs substances composites atteignant 100 % sans contaminants non volontaires comme composant biocéramique.

2. Utilisation d'un matériau céramique comme composant biocéramique selon la revendication 1, dans laquelle 20 à 60 % en volume de la matrice se présentent sous la forme de plaquettes hexagonales.

3. Utilisation d'un matériau céramique comme composant biocéramique selon la revendication 1 ou 2, dans laquelle 33 à 50 % en volume de la matrice se présentent sous la forme de plaquettes hexagonales.

4. Utilisation d'un matériau céramique comme composant biocéramique selon l'une des revendications précédentes, dans laquelle la quantité maximale d'yttria de stabilisation est de 1 à 2 % en moles.

5. Utilisation d'un matériau céramique comme composant biocéramique selon l'une des revendications précédentes, dans laquelle la quantité maximale d'yttria de stabilisation est de 1,5 à 1,8 % en moles.

6. Utilisation d'un matériau céramique comme composant biocéramique selon l'une des revendications précédentes, dans laquelle la quantité maximale de cérine de stabilisation est de 2,5 à 6 % en moles.

7. Utilisation d'un matériau céramique comme composant biocéramique selon l'une des revendications précédentes, dans laquelle la quantité maximale de cérine de stabilisation est de 3 à 5 % en moles.

8. Utilisation d'un matériau céramique comme composant biocéramique selon l'une des revendications précédentes, dans laquelle la quantité maximale de Pr₆O₁₁ est de 0,2 à 0,8 % en volume.

9. Utilisation d'un matériau céramique comme composant biocéramique selon l'une des revendications précédentes, dans laquelle la quantité maximale de Pr₆O₁₁ est de 0,25 à 0,5 % en volume.
